# EUROPÄISCHE PATENTSCHRIFT

(11) **EP 0 521 948 B1**
(45) Veröffentlichungstag und Bekanntmachung des Hinweises auf die Patenterteilung: **14.06.1995**
(21) Anmeldenummer: 91906466.7
(22) Anmeldetag: 22.03.1991
(51) Int. Cl.: C12N 9/10, C12P 19/18, C12N 15/54, C12N 1/21

(54) **$g(g)-CGTase**
$g(g)-CGTase
$g(g)-CGTase

(30) Priorität: 27.03.1990 DE 4009822
(43) Veröffentlichungstag der Anmeldung: 13.01.1993
(73) Patentinhaber: Consortium für elektrochemische Industrie GmbH, D-81379 München (DE)
(72) Erfinder: SCHMID, Gerhard, D-8000 München 60 (DE)
(86) Internationale Anmeldenummer: EP9100560
(87) Internationale Veröffentlichungsnummer: WO9114770

(56) Entgegenhaltungen:
- EP-A- 0 291 067
- Proc. Int.Symp. Cyclodextrins,4th,1988,87-92,Huber,O.;Szejtli,J.(Eds.)
- Chemical Abstract,Vol.106,1987,Columbus,Ohio,US;Kato,Takashi et al.:"A new gamma-cyclodextrin-forming enzyme produced by Bacillus subtilis No.313", see page 196,abstract 29122j & Denpun Kagaku 1986,33(2),137-43 (cited in the application).
- Agric.Biol.Chem.Vol.50,No.8,1986,Tokyo,JP.;T.Kato et al.:"Cloning and expression of the Bacillus subtilis No. 313 gamma-cyclodextrin forming CGTase gene in Escherichia coli",pages 2161-2162,see the whole article,especially figure 2(cited in the application).
- Chemical Abstract,Vol.113,22 October 1990 Columbus,Ohio,US;J.Hellman et al.:"Effects of modifications at the C-terminus of cyclomaltodextrins glucanotransferase from Bacillus circulans var.alkalophilus on catalytic activity",see page 349,abstract No.147978k & Biotechnol.Appl.Biochem.1990,12(4),387-96
- Chemical Abstracts,Vol.112,7 May 1990,Columbus,Ohio,US;G.Schmid et al.:"Selective complexing agents for the production of gamma-cyclodextrin",see page 140,abstract No.181767e & Proc.Int.Symp.Cyclodextrins,4th 1988,87-92 see lines 13-18
- Journal of Fermentation and Bioengineering,Vol 70,No.3,1990,Sulta,JP;Y.Fujita et al.:"Purification and properties of cyclodextrin glycosylytansferase from Bacillus sp.AL-6",pages 150-154,see abstract.

## Beschreibung

Die Erfindung betrifft primär γ-Cyclodextrin produzierende Cyclodextringlycosyltransferasen, die mit dem Substrat Stärke primär γ-Cyclodextrin und als Folgeprodukte nahezu ausschließlich cyclische Oligosaccharide bilden.

Das Enzym Cyclodextringlycosyltransferase (Abk.: CGTase) E.C.2.4.1.19 katalysiert die Bildung von Cyclodextrinen aus Stärke. Je nach Anzahl der Glucose-Einheiten, aus denen sich das Cyclodextrin (CD) zusammensetzt, unterscheidet man α-CD (6 Glucose-Einheiten), β-CD (7 Glucose-Einheiten) und γ-CD (8 Glucose-Einheiten).

Bisher sind 2 Typen der CGTase bekannt:
a) primär α-Cyclodextrin bildende CGTasen, auch α-CGTase genannt, wie z.B. die CGTase von Bacillus macerans (UK-Patent GB 2169 902), von Klebsiella pneumoniae (EP-A 220 714) und von Bacillus stearothermophilus (UK Patent GB 2169 902).
b) primär β-Cyclodextrin bildende CGTasen oder β-CGTase, wie z.B. die CGTase von Bacillus circulans (US 4,477,568), von Bacillus megaterium (US Patent 38 12 011), von Bacillus ohbensis (JP 74 124 285), von Micrococcus sp. (EP-A 017 242) und von alkalophilen Bacillus sp. (J. Gen. Microbiol. 1988, 134, 97-105; Appl. Microbiol. Biotechnol. 1987, 26, 149-153).

Hinsichtlich einer γ-CGTase gibt es bisher drei Hinweise in der Literatur:
a) Die Cyclodextringlycosyltransferase von Bacillus sp. produziert bei Zugabe von EtOH überwiegend eine Mischung aus β- und γ-Cyclodextrin (10,4 zu 18,7%) (JP 63,133,998). Das Enzym ist bezüglich seiner kinetischen Eigenschaften nicht charakterisiert, so daß es nicht einem speziellen Typ zugeordnet werden kann.
b) In drei Artikeln (Agric. Biol. Chem., 1986, 50 (8), 2161-2162 und Denpun Kagaku 1986, 33, 137, CA 106, 1987, 29122j) wird eine γ-CGTase von Bacillus subtilis No. 313 beschrieben. Diese CGTase zeichnet sich aus durch Bildung von γ-Cyclodextrin und linearen Oligosacchariden. Da CGTasen aus Stärke lediglich cyclische Produkte generieren, handelt es sich bei dieser "CGTase" um eine Übergangsform einer α-Amylase (erzeugt lineare Oligosaccharide aus Stärke) und einer CGTase. Diese "γ-CGTase" ist ungeeignet für die Herstellung von γ-Cyclodextrin, da nur geringe Ausbeuten erzielt werden können (siehe EP-A 327 099, 2. Seite, Zeile 40-43).
c) In "Proceedings of the Fourth International Symposium on Cyclodextrins, O.Huber und J.Szejtli (Eds.), Seiten 87-92, 1988" wird die Isolierung von 2 Bacilli-Stämmen beschrieben, die eine primär γ-Cyclodextrin bildende CGTase sekretieren. Die γ-CGTase aus Stamm 290-3 wurde isoliert. Das Korrespondierende Gen wurde Kloniert und in E.coli überexprimiert.

Aufgabe der Erfindung war es deshalb Enzyme, die überwiegend γ-CD produzieren sowie Verfahren zu ihrer Herstellung und Verwendung, zur Verfügung zu stellen.

Eine weitere Aufgabe der Erfindung war es, Expressionsplasmide und DNA-Sequenzen, die für oben genannte Enzyme kodieren, zur Verfügung zu stellen.

Die Aufgabe wird gelöst, durch eine γ-Cyclodextringlycosyltransferase, die dadurch gekennzeichnet ist, daß sie durch ein im 5' Bereich durch eine die homologen Aminosäuren der β-CGTase aus Bacillus 1-1 kodierende DNA-Region substituiertes Gen der γ-Cyclodextringlycosyltransferase erhältlich aus Bacillus 290-3 (DSM 5850) kodiert wird.

γ-Cyclodextringlycosyltransferasen im Sinne der Erfindung sind primär γ-Cyclodextrin produzierende γ-Cyclodextringlycosyltransferasen, welche mit dem Substrat Stärke primär γ-Cyclodextrin und als Folgeprodukte nahezu ausschließlich cyclische Oligosaccharide bilden.

Die erfindungsgemäßen CGTasen sind dadurch erhältlich, daß
a) alkalophile stärkeabbauende Bakterien auf die Sekretion einer γ-CGTase gescreent werden,
b) diese Bakterien taxonomisch charakterisiert werden
c) die γ-CGTase der Bakterien gereinigt und biochemisch charakterisiert wird,
d) das Gen der γ-CGTase kloniert und sequenziert wird,
e) dieses Gen in der den 5' Bereich des reifen Proteins kodierenden Region derart modifiziert wird, daß die den 5' Bereich kodierende Region durch eine den homologen Bereich einer anderen CGTase kodierende Region ausgetauscht wird,
f) das so modifizierte Gen unter die Kontrolle eines regulierbaren Promotors gestellt wird und in ein Bakterium so eingebracht wird, daß es mit diesem vermehrt wird und das Genprodukt sekretiert werden kann,
g) das Enzym aus dem Kulturüberstand nach Induktion und Expression gewonnen wird.

Zum Screenen der Bakterien auf die Produktion von γ-CGTase verwendet man bevorzugt stärkeabbauende, besonders bevorzugt alkalophile stärkeabbauende Bakterien.

Für die Charakterisierung der γ-CGTase produzierenden Bakterien wird die Form, Breite, Länge und Beweglichkeit der Bakterien bestimmt. Weiter wird ihre Färbbarkeit in der gram Reaktion, ihre Fähigkeit Katalase zu bilden sowie ihr GC-Gehalt ermittelt. Zusätzlich wird ihr Wachstumsverhalten bei verschiedenen Temperaturen, verschiedenen pH und verschiedenen NaCl Konzentrationen bestimmt.

Nach Reinigen der γ-CGTase wird das Enzym biochemisch charakterisiert. So werden beispielsweise Molekulargewicht, pH-Optimum, pH-Stabilität, Temperatur-Optimum und Temperatur-Stabilität des Enzyms bestimmt.

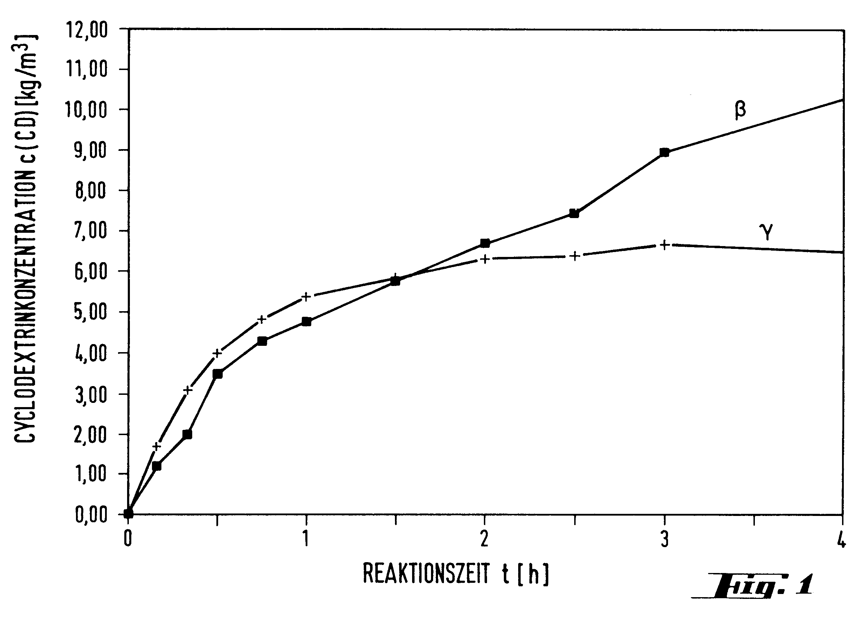

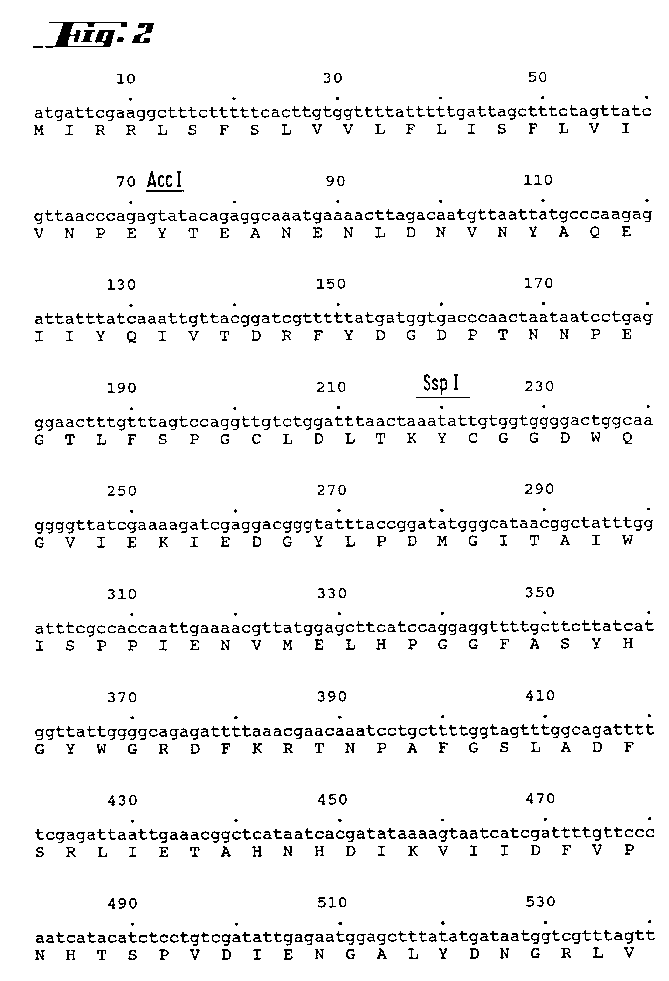

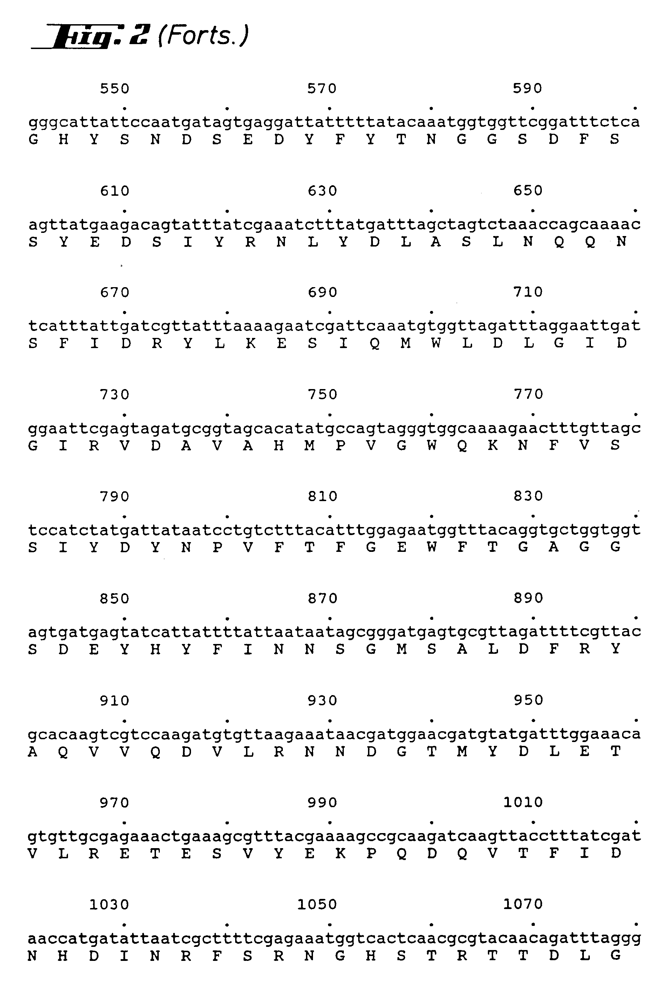

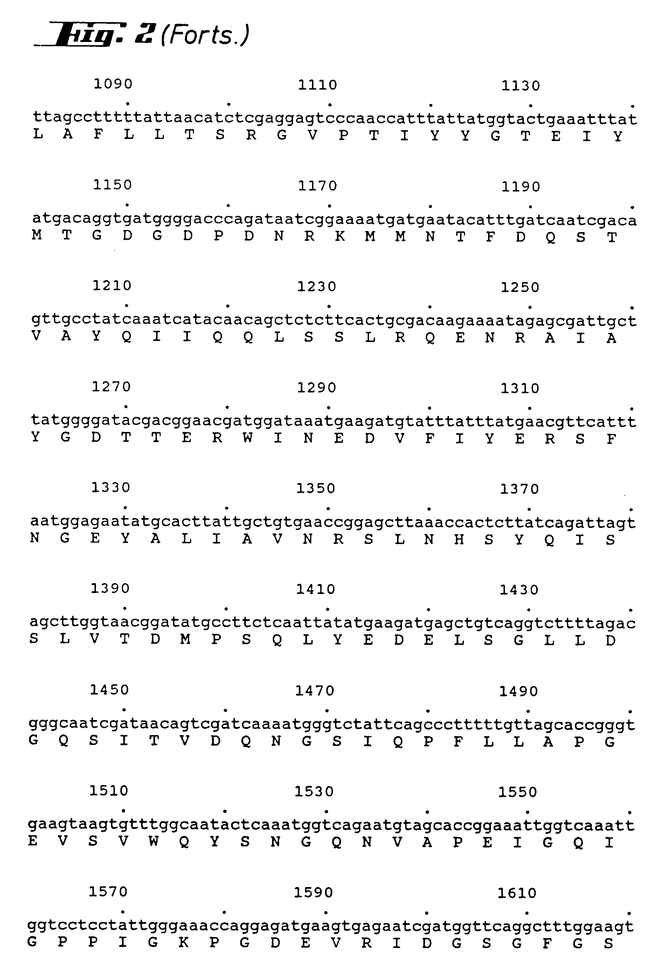

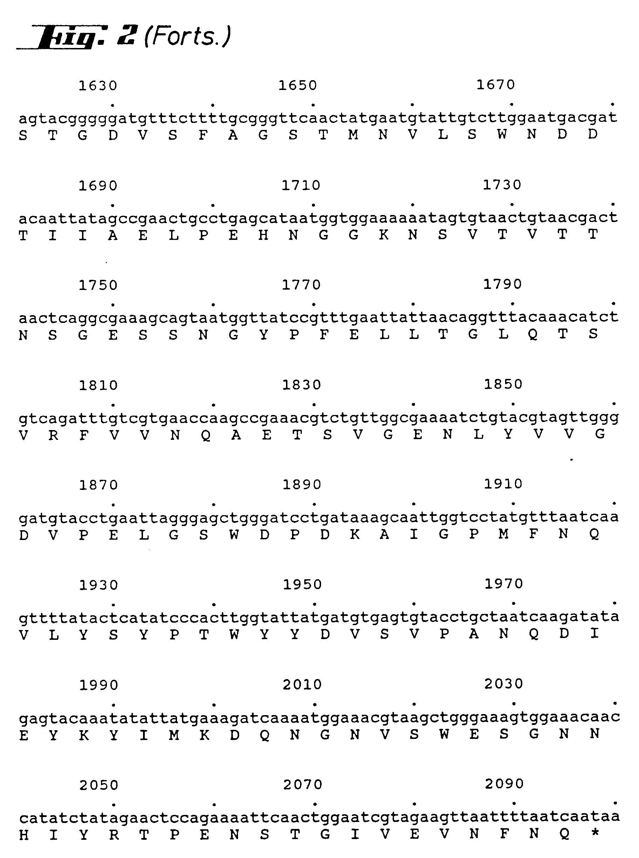

Um die Ausbeute an γ-CGTase zu steigern wird das kodierende Gen gentechnologisch modifiziert und in Sekretormutanten exprimiert. Dazu wird das Gen zunächst kloniert und sequenziert. Beispiele für erfindungsgemäße Gene sind die DNA kodierend eine Aminosäuresequenz gemäß Figur 2, DNA kodierend eine γ-CGTase enthaltend eine Aminosäuresequenz als Aminosäure 75 in Figur 2 oder enthaltend die in Figur 2 wiedergegebenen Aminosäurebereiche von Aminosäure 198 bis einschließlich Aminosäure 214 und/oder von Aminosäure 238 bis einschließlich Aminosäure 247 und/oder von Aminosäure 267 bis einschließlich Aminosäure 275 und/oder von Aminosäure 336 bis einschließlich Aminosäure 343. Bevorzugt wird das Gen in E. coli kloniert. Das identifizierte Gen wird dann unter die Kontrolle eines Promotors, bevorzugt eines regulierbaren Promotors (z.B. λP_{L}-, trp-, lac-, trc-Promotor), besonders bevorzugt des Lactose induzierbaren tac-Promotors, gestellt. Dadurch ist die steuerbare Überexpression der γ-CGTase möglich. Für eine optimale Sekretion ist die Verwendung eines Signalpeptids sinnvoll. Die Verwendung des eigenen Signalpeptids ist für die γ-CGTase Sekretion gut geeignet.

Die Erfindung betrifft somit ebenfalls Expressionsplasmide, die eine erfindungsgemäße Cyclodextringlycosyltransferase kodieren.

Ein Vektor, der neben dem die γ-CGTase kodierenden Gen und einer Leadersequenz, auch regulatorische Elemente, wie z.B. den tac-Promotor enthält, wird für die Produktion der γ-CGTase in Mikroorganismen, bevorzugt E. coli, besonders bevorzugt eine Sekretormutante von E. coli eingebracht. Geeignete Sekretormutanten lassen sich nach dem in EP-A-338 410 offenbarten Verfahren herstellen. Durch Induktion des Promotors, im Falle des tac-Promotors mit Lactose oder IPTG läßt sich die Überproduktion und Sekretion der γ-CGTase in das Kulturmedium erreichen. Aus dem Überstand der Sekretormutante läßt sich das erfindungsgemäße Protein in bekannter Weise aufreinigen.

Mit den hier beschriebenen Enzymen werden erstmals primär γ-Cyclodextrin produzierende CGTasen, die mit dem Substrat Stärke primär γ-Cyclodextrin und als Folgeprodukte nahezu ausschließlich cyclische Oligosaccharide bilden, hergestellt.

Die Enzyme lassen sich durch die beschriebenen gentechnischen Methoden in größeren Mengen gewinnen, als dies aus ihren natürlichen Ursprungsmikroorganismen möglich ist. Mit Hilfe der Enzyme lassen sich nach dem Verfahren, das in US-Patent 4,822,874 beschrieben ist, die Herstellungszeiten von γ-Cyclodextrinen stark verkürzen.

γ-Cyclodextrine erhöhen unter anderem die Löslichkeit von hydrophoben Substanzen in wäßriger Lösung, sie stabilisieren labile Substanzen (z.B. UV-Schutz, Oxidationsschutz) und binden flüchtige Substanzen.

γ-Cyclodextrin kann auch als Formulierungsmittel verwendet werden.
Die γ-Cyclodextrine finden u.a. Einsatz in folgenden Bereichen:
Pharmazeutische Industrie
Lebensmittelindustrie
Kosmetik
Pflanzenschutz
chemische Industrie.

In den Beispielen wird die Isolierung einer erfindungsgemäßen DNA, die Herstellung einer erfindungsgemäßen γ-CGTase, sowie die Überexpression der γ-CGTase aus dem alkalophilen Bakterium Bacillus 290-3 (DSM 5850, hinterlegt bei der DSM Deutsche Sammlung für Mikroorganismen und Zellkulturen am 19.3.90) beschrieben.
- Fig. 1:: Kinetik der Produktion von β- und γ-Cyclodextrin der γ-CGTase des alkalophilen Bakterienstammes Bacillus 290-3
- Fig. 2:: Offener Leserahmen der γ-CGTase aus Bacillus 290-3
- Fig. 3:: Das zu Überexpression der γ-CGTase verwendete tac-Promotor-Plasmid pJF118u
- Fig. 4:: Die synthetischen Oligonukleotide M8 und M9
- Fig. 5:: Das Expressionsplasmid pCM750
- Fig. 6:: Die synthetischen Oligonukleotide M10, M11, M12 und M13
- Fig. 7:: Das Plasmid pCM720

### Beispiel 1: Screening nach γ-CGTase produzierenden alkalophilen Bakterien

Bodenproben aus verschiedensten Regionen der Erde wurden gesammelt. 0,1 - 0,2 g Erde wurden abgewogen und in sterilen Gefäßen mit 1 ml steriler physiologischer Kochsalzlösung aufgeschlämmt. Nach Absitzen der Grobanteile wurden jeweils 0,1 ml auf eine Stärkeagarplatte (Medium 1 : 10 g/l lösliche Stärke; 5 g/l Pepton; 5 g/l Hefeextrakt; 1 g/l KH₂PO₄; 0,2 g/l MgSO₄ x 7 H₂O; 10 g/l Na₂CO₃; 15 g/l Agar; pH 10,4) plattiert. Die Inkubation der Agarplatten erfolgte bei 30°C für 2 - 3 Tage. Kolonien von stärkeabbauenden Bakterien zeigten einen trüben Halo, der durch Retrogradierung niedermolekularer Stärkemoleküle entstand. Die Kolonien wurden isoliert und zweimal auf Stärkeagarplatten gereinigt. Daraufhin wurde eine Anzucht in 2 ml Flüssigmedium obiger Zusammensetzung durchgeführt. Nach 48 h Inkubation bei 30°C wurden die Zellen abzentrifugiert und der Überstand auf CGTase-Aktivität getestet. 200 »l Überstand wurden mit 200 »l 10 %iger Stärkelösung in 20 mM Tris/HCl pH 9,0; 5 mM CaCl₂ 1 - 5 h bei 40°C inkubiert. Die Enzymreaktion wurde durch Zugabe von 600 »l Methanol gestoppt und der Überstand nach Zentrifugation mittels HPLC analysiert. Aus einer Vielzahl von Isolaten wurde der Stamm Bacillus 290-3 isoliert, der eine CGTase ins Kulturmedium ausscheidet, die kinetisch bevorzugt γ-Cyclodextrin bildet (Fig. 1).

### Beispiel 2: Taxonomische Charakterisierung des Stamms Bacillus 290-3

Die taxonomische Einordnung ergab, daß es sich um ein gram positives, sporenbildendes Bakterium handelt, das dem bisher nicht exakt charakterisierten Bacillus firmus/lentus-Komplex zuzuordnen ist (siehe Tabelle 1).

**Tabelle 1**

| Taxonomische Merkmale des Isolats 290-3 | | |
|---|---|---|
| Eigenschaften | | Bacillus 290-3 |
| Stäbchen | | + |
| Breite »m | | 0.7-0.9 |
| Länge »m | | 2.5-4.0 |
| Beweglichkeit | | + |
| Endosporen | | + |
| Gram Reaktion | | + |
| Katalase | | + |

| Maximale Temperatur | | |
|---|---|---|
| Wachstum positiv bei °C | | 40 |
| Wachstum negativ bei °C | | 45 |

| Wachstum in | | |
|---|---|---|
| Medium pH 5.7 | | - |
| Medium pH 7.0 | | - |
| NaCl | 5 % | + |
| | 7 % | + |
| mol % G+C | | 36,6 |

### Beispiel 3: Reinigung der γ-CGTase

Der Stamm Bacillus 290-3 wurde in Medium 1 (siehe Beispiel 1) angezogen. Nach einer Wachstumsdauer von 48 h wurden die Zellen durch Zentrifugation abgetrennt und dem Kulturüberstand (NH₄)₂SO₄ zugegeben bis eine Sättigung von 66 % erreicht war. Die Mischung wurde 1 Stunde bei 4°C aufbewahrt und daraufhin das Präzipitat durch Zentrifugation (10 000 x g; 20 min) abgetrennt. Das Präzipitat wurde in 1/100 des Ausgangsvolumens in Puffer A (20 mM Tris/Cl pH 8,5, 5 mM CaCl₂, 0.05 % γ-CD) resuspendiert und gegen denselben Puffer dialysiert. Nach Zentrifugation (10 min; 10 000 x g) wurde die Enzymlösung auf eine Affinitätssäule (γ-Cyclodextrin gekoppelt an Sepharose 6B über Butyldiglycidyläther) aufgetragen. Die Elution erfolgte mit Puffer A, der mit 1 % γ-Cyclodextrin versehen wurde.

Das eluierte Proteinmaterial wurde durch Ammoniumsulfat-Fällung konzentriert und erneut dialysiert. Die Reinheit des Proteins wurde durch SDS-Polyacrylamidgelelektrophorese kontrolliert.

### Beispiel 4: Biochemische Charakterisierung der γ-CGTase

Die biochemische Charakterisierung der γ-CGTase führte zu folgenden Ergebnissen:
Molekulargewicht 75 000 Da
pH-Optimum 6.0 - 8.0
pH-Stabilität 5.0 - 10.0
Temp.-Optimum 60°C
Temp.-Stabilität bis 50°C

### Beispiel 5: Klonierung und Sequenzierung des Gens für die γ-CGTase

### Klonierung:

Chromosomale DNA des Stammes Bacillus 290-3 wurde partiell mit dem Restriktionsenzym Sau 3A gespalten und nach Größenfraktionierung der Fragmente über Agarosegelelektrophorese wurden diese DNA-Bruchstücke in pUC18 (BamHI gespalten) kloniert.

2 x 10⁴ Klone dieser Genbank wurden mit einem radioaktiv markierten, 1,6Kb großen DNA-Fragment (BglII-PstI) des kodierenden Bereichs der β-CGTase von Bacillus 1-1 (Proc. 4th Int. Symposium on Cyclodextrins (Huber O., Szejtli J., eds) 1988, pp. 71-76, Kluwer Academic) in einem Koloniehybridisierungsversuch (Maniatis et al. 1982, Cold Spring Harbor Laboratory) analysiert. Dabei wurden die Klone p19 und p43 identifiziert, deren Plasmide das Gen der γ-CGTase beinhalteten.

### Sequenzierung:

Zur Bestimmung der Nukleotidsequenz des γ-CGTase-Strukturgens wurden DNA-Fragmente in das Plasmid pUC18 oder pUC19 subkloniert. Mit Exonuclease III wurden Deletionen in den Inserts dieser Plasmide so generiert, daß die DNA-Sequenzierung nach der "Sanger-Dideoxy-Kettenabbruchmethode" zu überlappenden Sequenzen führte (DNA, 1985, 4, 165-170). Der offene Leserahmen, der für die γ-CGTase kodiert, umfaßt 2097 Nukleotide (Fig. 2). Das daraus abgeleitete Protein besteht aus 699 Aminosäuren mit einem Molekulargewicht von 78 000 Da. Nach Abspaltung des Signalpeptides ergibt sich ein Molekulargewicht von 75 000 Da.

### Beispiel 6: Expression und Sekretion der γ-CGTase in E. coli

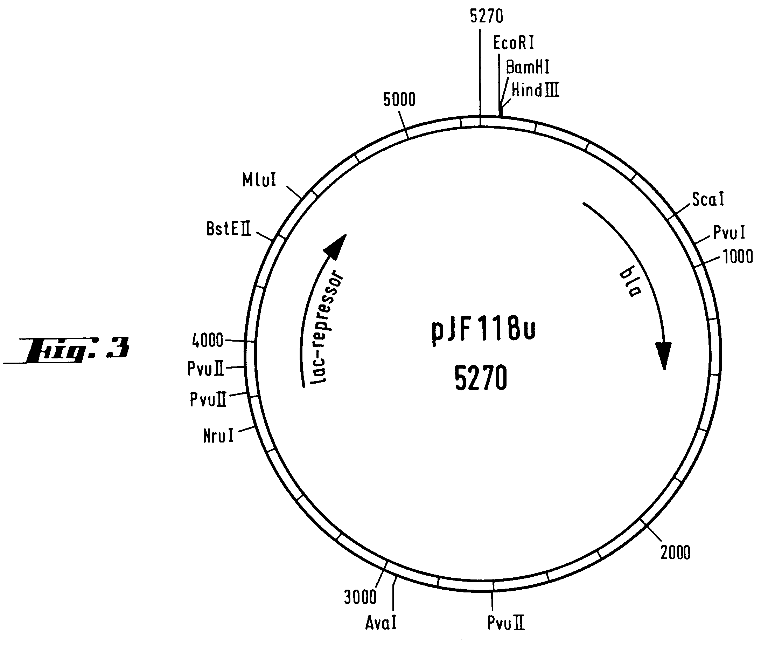

Für die Überexpression der γ-CGTase wurde das "tac"-Promotorplasmid pJF118u (Fig. 3) verwendet. Das Plasmid wurde mit den Restriktionsenzymen EcoRI und HindIII gespalten und über Agarosegelelektrophorese wurde das kleine DNA-Fragment aus dem "poly-linker" abgetrennt.

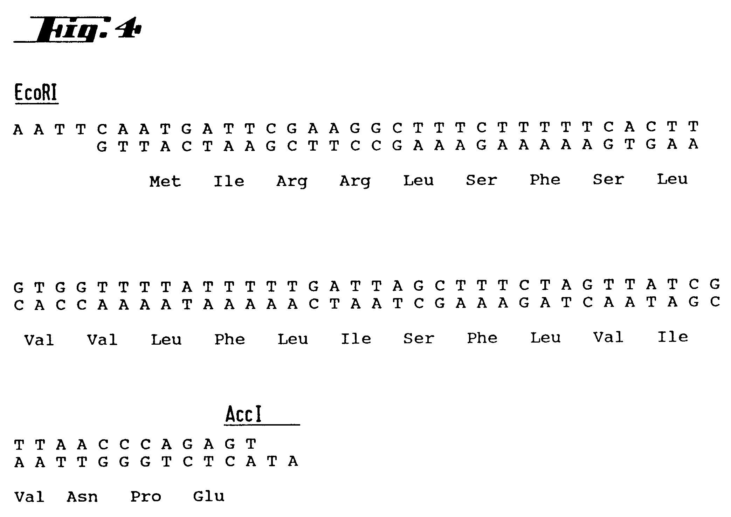

Das Plasmid p19 (siehe Beispiel 5) wurde mit AccI und HindIII gespalten und über Agarosegelelektrophorese wurde ein 2,4 kb großes DNA-Fragment isoliert, das das Strukturgen der γ-CGTase nahezu vollständig trägt. Es fehlt am 5' Ende des Gens (siehe "AccI-site" in Fig. 2) eine kurze für das Signalpeptid kodierende Region. Durch zwei synthetische Oligonukleotide M8 und M9 (Fig. 4) wird diese Region ersetzt.

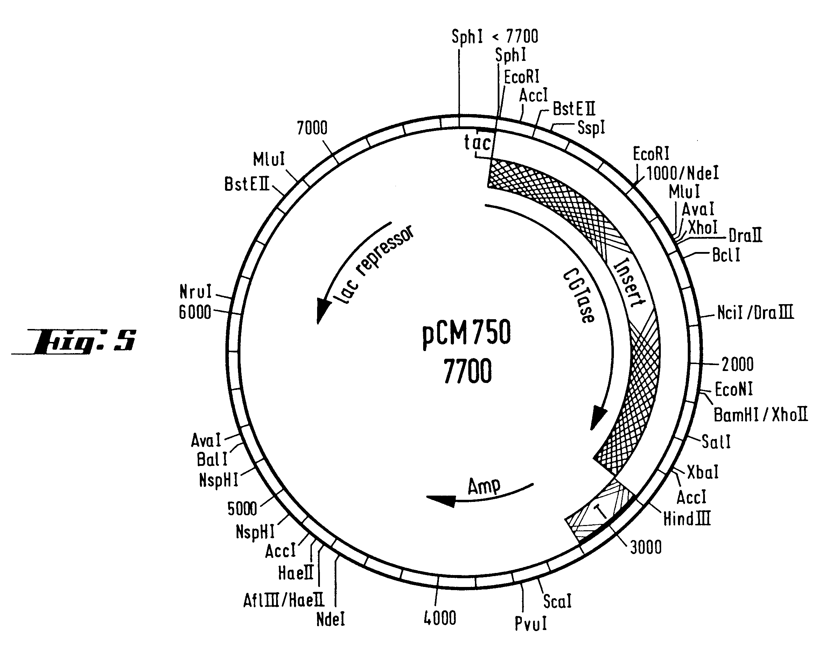

Durch Ligation des EcoRI-HindIII-Fragmentes von pJF118u mit dem Oligonukleotidpaar M8, M9 und dem AccI-HindIII Fragment von p19 entstand das Expressionsplasmid pCM750 (Fig. 5).

Transformation der Sekretormutante E. coli WCM100 mit dem Plasmid pCM750 und Anzucht des transformierten Stammes in Vollmedium (10 g/l Pepton; 5 g/l Hefeextrakt; 5 g/l NaCl; 10 g/l Lactose; 0,1 g/l CaCl₂; 100 mg/l Ampicillin) bei 30°C ermöglichte eine 500fach höhere γ-CGTase-Ausbeute im Vergleich zu den Enzymausbeuten mit dem Stamm Bacillus 290-3.

### Beispiel 7: Modifizierung der γ-CGTase

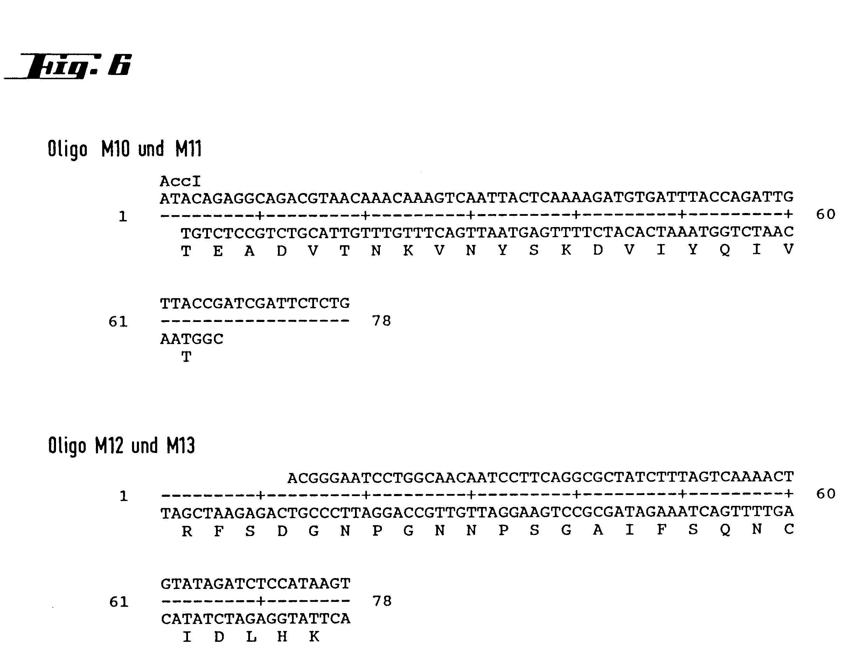

Nach dem Phosphoramidit-Verfahren wurden die Oligonukleotidpaare M10, M11 und M12, M13 synthetisiert (Fig. 6). Die DNA-Sequenz der Oligonukleotide kodiert für die N-terminale Aminosäuresequenz der β-CGTase von Bacillus 1-1 (Proc. 4th Int. Symposium on Cyclodextrins (Huber O., Szejtly J., eds) 1988, pp 71-76, Kluwer Academic) und ist homolog zum N-terminalen Bereich der γ-CGTase, der die DNA-Region bis zur SspI-Schnittstelle umfaßt (siehe Fig. 2).

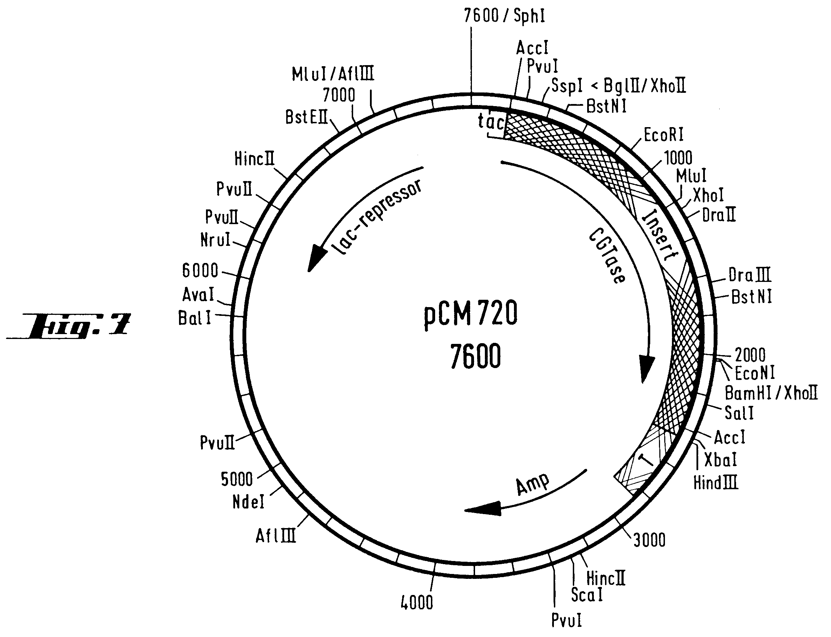

Das Plasmid pCM750 wurde partiell mit dem Restriktionsenzym AccI gespalten. Mit SspI wurde nachgespalten und über Agarosegelelektrophorese wurde ein DNA-Fragment der Größe 7,3 kb isoliert. Die Ligation dieses DNA-Fragments mit den doppelsträngigen Oligonukleotiden M10, M11 und M12, M13 ergab das Plasmid pCM720 (Fig. 7), das für eine chimäre γ-CGTase kodiert.

Transformation der Sekretormutante E. coli WCM100 und Anzucht entsprechend Beispiel 6 erbrachte eine zweifache Steigerung der γ-CGTase-Ausbeute im Vergleich zu den Ergebnissen von Beispiel 6.

### Beispiel 8: Herstellung von γ-Cyclodextrin mit γ-CGTase

10 g lösliche Stärke wurden in 100 ml Puffer (10 mmol/l Tris/HCl pH 8.0 und 5 mM CaCl₂) aufgenommen und die Stärke durch Erhitzen für 5 min auf 95°C gelöst. Nach dem Abkühlen auf 50°C wurde als selektiver Komplexbildner für γ-Cyclodextrin 1 g Cyclohexadecenon zugegeben. Daraufhin wurden 100 U γ-CGTase zupipettiert. Der Ansatz wurde kräftig gerührt und die Reaktionstemperatur wurde bei 50°C gehalten. Die optimale Ausbeute an γ-Cyclodextrin von 48 % bezogen auf die eingesetzte Stärke wurde nach 8 Stunden Reaktionszeit erreicht.

### Vergleichsbeispiel:

Der Versuch wurde durchgeführt wie in Beispiel 5 beschrieben mit der Ausnahme, daß statt γ-CGTase 100 U α-CGTase von Bacillus macerans verwendet wurde. Nach 32 Stunden wurde eine maximale Ausbeute an γ-Cyclodextrin von 43 % erreicht.

## Patentansprüche

1. γ-Cyclodextringlycosyltransferase, dadurch gekennzeichnet, daß sie durch ein im 5' Bereich durch eine die homologen Aminosäuren der β-CGTase aus Bacillus 1-1 kodierende DNA-Region substituiertes Gen der γ-Cyclodextringlycosyltransferase erhältlich aus Bacillus 290-3 (DSM 5850) kodiert wird.

2. Expressionsplasmide, die eine Cyclodextringlycosyltransferase nach Anspruch 1 kodieren.

3. Verfahren zur Gewinnung von γ-Cyclodextringlycosyltransferasen, dadurch gekennzeichnet, daß
a) alkalophile stärkeabbauende Bakterien auf die Sekretion einer γ-CGTase gescreent werden,
b) diese Bakterien taxonomisch charakterisiert werden
c) die γ-CGTase der Bakterien gereinigt und biochemisch charakterisiert wird,
d) das Gen der γ-CGTase kloniert und sequenziert wird,
e) dieses Gen in der den 5' Bereich des reifen Proteins kodierenden Region derart modifiziert wird, daß die den 5' Bereich kodierende Region durch eine den homologen Bereich einer anderen CGTase kodierende Region ausgetauscht wird,
f) das so modifizierte Gen unter die Kontrolle eines regulierbaren Promotors gestellt wird und in ein Bakterium so eingebracht wird, daß es mit diesem vermehrt wird und das Genprodukt sekretiert werden kann,
g) das Enzym aus dem Kulturüberstand nach Induktion und Expression gewonnen wird.

4. Verfahren nach Anspruch 3, dadurch gekennzeichnet, daß als die den homologen Bereich einer anderen CGTase kodierende Region eine den entsprechenden Bereich aus Bacillus 1-1 kodierende Region verwendet wird.

5. DNA, kodierend eine Aminosäuresequenz gemäß Figur 2.

6. DNA, kodierend eine γ-Cyclodextringlycosyltransferase, enthaltend eine Aminosäuresequenz ab Aminosäure 75 in Figur 2.

7. DNA, kodierend eine γ-Cyclodextringlycosyltransferase, enthaltend die in Figur 2 wiedergegebenen Aminosäurebereiche von Aminosäure 198 bis einschließlich Aminosäure 214 und/oder von Aminosäure 238 bis einschließlich Aminosäure 247 und/oder von Aminosäure 267 bis einschließlich Aminosäure 275 und/oder von Aminosäure 336 bis einschließlich Aminosäure 343.

8. Verfahren zur Herstellung von γ-Cyclodextrin, dadurch gekennzeichnet, daß eine γ-CGTase nach Anspruch 1 eingesetzt wird.

9. Bakterienstamm Bacillus 290-3 (DSM 5850).

## Claims

1. γ-Cyclodextringlycosyltransferase, characterized in that it is encoded by a gene of γ-cyclodextringlycosyltransferase obtainable from Bacillus 290-3 (DSM 5850), which is substituted in the 5' region by a DNA region encoding the homologous amino acids of the β-CGTase from Bacillus 1-1.

2. Expression plasmids which encode a cyclodextrin glycosyltransferase according to Claim 1.

3. Process for obtaining γ-cyclodextrin glycosyltransferases, characterized in that
a) alkalophilic starch-degrading bacteria are screened for the secretion of a γ-CGTase,
b) these bacteria are characterized taxonomically,
c) the γ-CGTase from the bacteria is purified and biochemically characterized,
d) the γ-CGTase gene is cloned and sequenced,
e) this gene is modified in the region encoding the 5' region of the mature protein such that the region encoding the 5' region is replaced by a region encoding the homologous region of another CGTase,
f) the gene modified in this way is placed under the control of a regulatable promoter and introduced into a bacterium such that it is replicated by this and the gene product can be secreted,
g) the enzyme is obtained from the culture supernatant after induction and expression.

4. Process according to Claim 3, characterized in that as the region encoding the homologous region of another CGTase a region encoding the corresponding region of Bacillus 1-1 is used.

5. DNA, encoding an amino acid sequence as in Figure 2.

6. DNA, encoding a γ-cyclodextringlycosyltransferase containing an amino acid sequence from amino acid 75 in Figure 2.

7. DNA, encoding a γ-cyclodextringlycosyltransferase containing the amino acid regions shown in Figure 2 from amino acid 198 up to and including amino acid 214 and/or from amino acid 238 up to and including amino acid 247 and/or from amino acid 267 up to and including amino acid 275 and/or from amino acid 336 up to and including amino acid 343.

8. Process for preparing γ-cyclodextrin, characterized in that a γ-CGTase according to Claim 1 is employed.

9. Bacterial strain Bacillus 290-3 (DSM 5850).

## Revendications

1. γ-cyclodextrine-glycosyltransférase, caractérisée en ce qu'elle est codée par un gène substitué dans le domaine 5' par une région d'ADN qui code pour les acides aminés homologues de la β-CGTase du Bacillus 1-1, gène de la γ-cyclodextrine-glycosyltransférase que l'on peut obtenir à partir du Bacillus 290-3 (DSM 5850).

2. Plasmides d'expression qui codent pour une cyclodextrine-glycosyltransférase selon la revendication 1.

3. Procédé pour la préparation de γ-cyclodextrine-glycosyltransférases caractérisé en ce que :
a) on crible des bactéries alcalophiles qui dégradent l'amidon pour détecter la sécrétion d'une γ-CGTase,
b) on caractérise taxonomiquement ces bactéries,
c) on purifie la γ-CGTase des bactéries et on la caractérise biochimiquement,
d) on clone et on séquence le gène de la γ-CGTase,
e) on modifie ce gène dans la région qui code pour le domaine 5' de la protéine mature de telle façon que la région qui code pour le domaine 5' est substituée par une région qui code pour le domaine homologue d'une autre CGTase,
f) on met sous le contrôle d'un promoteur régulable le gène ainsi modifié et on le transfère dans une bactérie de telle façon qu'il se multiplie dans celle-ci et qu'il sécréte le produit du gène,
g) on récupère l'enzyme à partir du surnageant après induction et expression.

4. Procédé selon la revendication 3, caractérisé en ce qu'on utilise en tant que région codante pour le domaine homologue d'une autre CGTase, une région codante pour le domaine correspondant à partir de Bacillus 1-1.

5. ADN codant pour une séquence d'acides aminés selon la figure 2.

6. ADN codant pour une γ-cyclodextrine-glycosyltransférase contenant une séquence d'acides aminés à partir de l'acide aminé 75 dans la figure 2.

7. ADN codant pour une γ-cyclodextrine-glycosyltransférase contenant les domaines d'acides aminés reproduits sur la figure 2 à partir de l'acide aminé 198 à y compris l'acide aminé 214 et/ou de l'acide aminé 238 à y compris l'acide aminé 247 et/ou de l'acide aminé 267 à y compris l'acide aminé 275 et/ou de l'acide aminé 336 à y compris l'acide aminé 343.

8. Procédé pour la préparation de γ-cyclodextrine, caractérisé en ce qu'on utilise une γ-CGTase selon la revendication 1.

9. Souche de bactérie Bacillus 290-3 (DSM 5850).
